(19) Europäisches Patentamt
European Patent Office
Office européen des brevets

(11) **EP 4 216 926 B1**

(12) **EUROPEAN PATENT SPECIFICATION**

(45) Date of publication and mention
of the grant of the patent:
**04.03.2026 Bulletin 2026/10**

(21) Application number: **21790347.5**

(22) Date of filing: **21.09.2021**

(51) International Patent Classification (IPC):
*A61P 35/00* (2006.01)     *A61P 13/08* (2006.01)
*A61K 9/00* (2006.01)      *A61K 47/22* (2006.01)
*A61K 31/337* (2006.01)    *A61K 31/436* (2006.01)
*A61K 47/34* (2017.01)

(52) Cooperative Patent Classification (CPC):
**A61K 9/0024; A61K 31/337; A61K 31/436;
A61K 47/22; A61K 47/34; A61P 13/08**

(86) International application number:
**PCT/US2021/071538**

(87) International publication number:
**WO 2022/067309 (31.03.2022 Gazette 2022/13)**

(54) **SYSTEM AND METHOD FOR PROSTATE TREATMENT**

SYSTEM UND VERFAHREN ZUR PROSTATABEHANDLUNG

SYSTÈME ET PROCÉDÉ DE TRAITEMENT DE LA PROSTATE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.09.2020 US 202063081865 P
06.11.2020 US 202017092079**

(43) Date of publication of application:
**02.08.2023 Bulletin 2023/31**

(73) Proprietor: **Resurge Therapeutics, Inc.
San Jose, CA 95124 (US)**

(72) Inventors:
• **TROLLSAS, Olof, Mikael
San Jose, CA 95124 (US)**
• **STANKUS, John, J.
Santa Cruz, CA 95062 (US)**
• **GHOLAMI, Shahram, Shawn
Monte Sereno, CA 95030 (US)**

(74) Representative: **Snaith, James Michael et al
Kilburn & Strode LLP
Lacon London
84 Theobalds Road
London WC1X 8NL (GB)**

(56) References cited:
**WO-A1-2014/066085      WO-A1-2016/013829
WO-A1-2018/128173      US-A1- 2008 194 663
US-B1- 6 277 391**

• SERGEY SHIKANOV ET AL: "Intratumoral
Delivery of Paclitaxel for Treatment of Orthotopic
Prostate Cancer", JOURNAL OF
PHARMACEUTICAL SCIENCES, vol. 98, no. 3, 1
March 2009 (2009-03-01), US, pages 1005 - 1014,
XP055509946, ISSN: 0022-3549, DOI: 10.1002/
jps.21492

**Description**

FIELD

Minimally invasive, local treatments for men's health and, more particularly, lower urinary tract symptoms.

PRIORITY CLAIM

This application claims priority to U.S. provisional application no. 63/081,865, filed Sept. 22, 2020 (RTPROV-2), and U.S. non-provisional no. 17/092,079, filed Nov. 6, 2020 (RTNPROV-1).

BACKGROUND

Benign Prostatic Hyperplasia (BPH) is a noncancerous increase in size of the prostate gland due to proliferation of glandular epithelial tissue, smooth muscle and connective tissue within the prostate transition zone that causes lower urinary tract symptoms. Lower urinary tract symptoms (LUTS) include voiding or obstructive symptoms such as hesitancy, poor and/or intermittent stream, straining, feeling of incomplete bladder emptying, and storage or irritative symptoms such as frequency, urgency, urge incontinence, and nocturia. It affects approximately half of men aged 50 and over and by age 80, 90% of men are affected. Treatment options consist of lifestyle changes, medications, various procedures, and surgery. Lifestyle changes consist of weight loss, exercise, and decreased caffeine consumption. With more significant symptoms, oral medications such as alpha blockers (e.g. terazosin) or 5alpha-reductase inhibitors (e.g. finasteride) are prescribed. These medications, requiring daily dosing for patient compliance, may require a long onset to show efficacy, if at all, and carry side effects such as ejaculation changes, erectile dysfunction, weakness, headaches, and decreased libido.

There is an unmet clinical need to treat BPH with improved and sustained efficacy, administered via a less invasive procedure and with less associated side effects.

US6277391B1 in accordance with its abstract states "This invention relates to a composition *and method for treating diseases and disorders of the prostate such as prostatitis, benign prostatic hypertrophy, and prostate carcinoma. The prostate is treated by intraprostatic injection of a biodegradable sustained release formulation. By injecting the treatment substance directly into the prostate, improved treatment results are obtained with a much lower treatment substance dosage. Additionally, by incorporating the treatment substance into a biodegradable sustained release formulation, the need for frequent repetition of injections is eliminated".*

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

**SUMMARY**

According to an aspect as defined in claim 1, there is provided a sustained release formulation (SRF) for use in a method of treatment of benign prostatic hyperplasia (BPH), the SRF comprising a cytostatic or cytotoxic drug,

wherein the method comprises dispensing the SRF into the target tissue of the prostate and wherein dispensing comprises 10-500 microliter per injection over 1-10 injections, across each side of the prostate.

In view of the foregoing, also described but not presently claimed herein is an apparatus, method, and system for minimally invasive treatment of BPH, with sustained efficacy and less adverse side effects using a sustained release formulation.

The disclosure is generally directed to achieving a local delivery of a concentration of a sustained efficacy treatment to a target tissue associated with the prostate, and/or providing relief of urinary tract symptoms originating from, or associated with an enlarged prostate while mitigating if not avoiding damage to nearby prostate structures or the urethra. The treatment may be used by itself, or in combination with other known treatments.

Accordingly, a treatment of prostatic hyperplasia tissue, as provided herein, includes the delivery of a drug or multiple drugs to the tissue in a sustained release manner via the sustained release formulation. The treatment may be used with, or in addition to treatments involving removal of tissue, and/or delivery of energy to the tissue and additionally the administering of various agents.

A treatment for BPH according to the disclosure may include, for example, a mechanical treatment of the tissue (e.g., stenting, ballooning, thermal ablation, lasing, surgery), or delivery of pharmaceutical, biologic or chemical agents, drugs, including pharmaceutical, biologic, or chemical agents that may be delivered locally along with, or complimented with introduction of the sustained release formulation into the body. The sustained release formulation may additionally, or alternatively, be administered after a treatment of BPH according to other methods.

Access to prostatic tissue may be achieved transurethrally, transrectally or transperineally via an existing body orifice. It may be beneficial and less invasive to access the tissue by either transrectal or transperineal approaches. The advantages with a transrectal or transperineal approach include one or more (1) oral and/or local anesthesia application instead of general anesthesia, (2) less trauma to the urethra tract and less resulting side effects also reducing the need for catheterization, (3) faster recovery time for the patient, (4) familiar treatment for the urologist physician similar to prostate cancer biopsy. For access by transrectal or transperineal approach, guidance may be provided by ultrasound, x-ray, computed tomography, magnetic resonance imaging or other imaging modality. Ultrasound imaging may be beneficial given that ultrasound is utilized for prostate biopsy. The transrectal approach closely mirrors the present prostate ultrasound and biopsy techniques familiar to urologists. Transrectal and transperineal approaches both avoid interaction with the urethra, which limits the caustic effects of urethral procedures therefore minimizing side effects and dysuria associated with currently available BPH procedures.

Also described but not presently claimed herein is a delivery vehicle and sustained release formulation for delivery of a drug or a combination of drugs in an efficacious manner for treatment of the prostate. The drug may be an anti-inflammatory, anti-proliferative, cytoreductive, cytostatic, and/or cytotoxic that would affect the prostate size and gland proliferation. This delivery vehicle enables delivery of one or more drugs into the target tissue. Once delivered to the target tissue, the drug may then release in a slow, sustained release fashion, optionally delivered as an initial burst of the drug, followed by a slow, sustained release of the drug to the target release. As will be appreciated, the amount or lack of burst and/or the "slow, sustained release" release period will depend on the drug delivered to the prostate. In some embodiments a slow, sustained release may occur over, e.g., a 24-hour period, 3 - 7 days, 1 - 4 weeks, 1 to 12 months, 3 months, or 6 months.

Also described but not presently claimed herein is a system for treating BPH including a delivery vehicle, the sustained release formulation, and imaging device for locating a target tissue of the prostate.

Also described but not presently claimed herein is an apparatus for treating BPH including a delivery vehicle adapted for being introduced into the body to introduce the sustained release formulation in liquid form to the tissue target.

Also described but not presently claimed herein is a method for treating BPH including injecting in or near the target tissue the sustained release formulation using a delivery vehicle.

Also described but not presently claimed herein is a sustained release formulation for treating BPH and deliverable to the targeted tissue using a needle or catheter, including a composition comprising the sustained release formulation.

Also described but not presently claimed herein is a system, apparatus and method adapted for treatment of BPH by a needle injection of a SRF at a target tissue, the benefits of which may include one or more of a less invasive procedure leading to greater patient acceptance and less complications during patient treatment, less frequent procedures needed, and less risk of an administered drug or treatment producing adverse consequences for urinary or sexual function.

It is appreciated that the SRF for treating BPH and deliverable to the targeted tissue using an extended needle or catheter, including a composition comprising the SRF, that has a viscosity allowing effective delivery (e.g., fully dispensed at the target tissue by a health care professional without backflow or excessive pressure being applied to inject the composition) through the needle or catheter, e.g. dynamic viscosity of 1-200 Centipoise (cP) (0.001-0.2 Pa.s) and gelation dynamic viscosity greater than 200 cP (0.2 Pa.s). At the same time the formulation needs to solidify or gel at a rate that allows the formulation to stay in the target tissue when the delivery system is removed.

It is appreciated that the total volume of the SRF for treating BPH and deliverable to the targeted tissue using an extended needle or catheter, including a composition comprising the SRF needs to be small enough to avoid causing additional acute pressure on the urethra, which may cause additional discomfort for the patients immediately after on shortly after the procedure. Other considerations include using a SRF that does not swell significantly due to uptake of fluids from the surrounding tissue. Additionally, avoidance of high pressure may be warranted to avoid backflow of the composition comprising the SRF through the delivery device (e.g., needle or catheter) and/or inhibiting the composition comprising the SRF from being expelled from the target tissue due to pressure buildup.

BRIEF DESCRIPTION OF THE DRAWINGS

FIG. 1 shows Drug Release Curves for Sustained Release Formulations (SRFs) A, B and C.

FIG. 2 shows gross pathology images of trimmed and formalin fixed treated prostate gland from a canine study acute animal 20C0083. Visible white material demonstrates successful local injection of an SRF2.

FIG. 3 shows an acute animal 20C0083 treated prostate histopathology H&E stained image with poorly staining injectate material (containing SRF2) in the left lateral lobe (asterisk). (U = urethra, arrows designate needle tracks).

FIG. 4 shows a chronic 30-day animal 20C0081 histopathology H&E stained image demonstrating degenerate smooth muscle in the left sided cranial prostate subsample (arrows). (U = urethra).

FIG. 5 shows a chronic 30-day animal 20C0081 histopathology H&E stained image demonstrating prostate tissue surrounding injectate demonstrating loss of adjacent glandular acini adjacent (arrow).

FIG. 6A shows glandular acini of the target tissue of the acute animal 20C0083 (histopathology H&E stained).

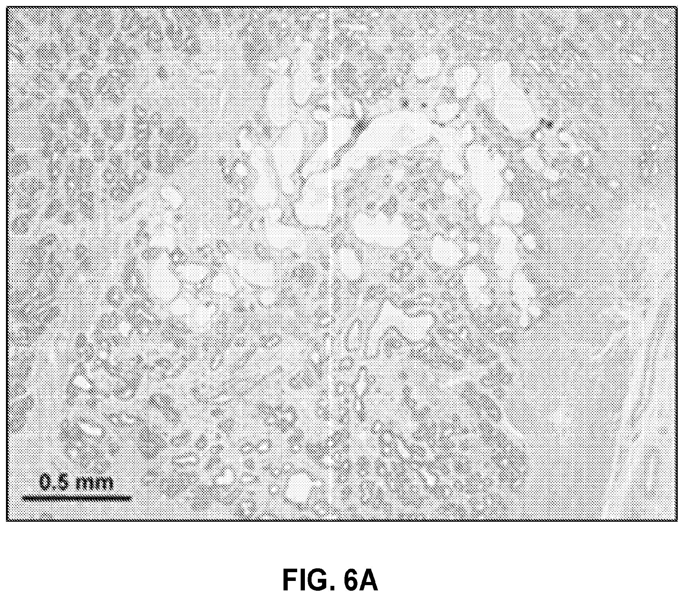

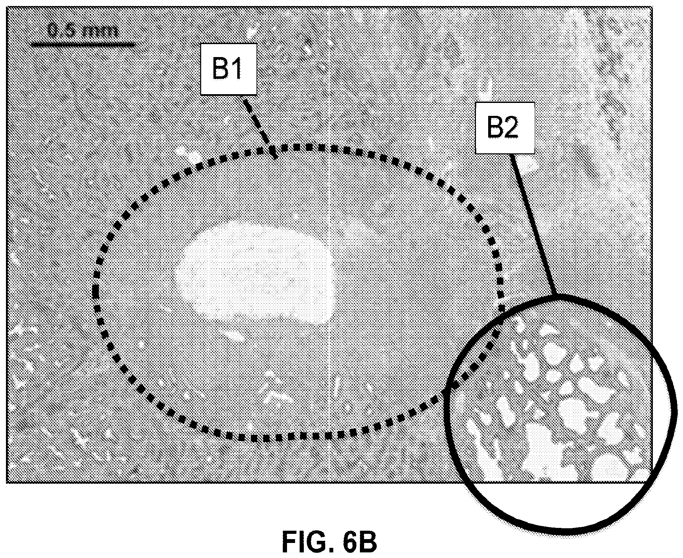

FIG. 6B shows the target tissue of the chronic 30-day animal 20C0081 (Masson's trichrome stained). The white area in the image is the location where the SRF was injected into the target tissue. Comparing FIGS. 6A to 6B, there is a loss of the glandular acini in the 30-day animal versus shown in the acute animal (FIG. 6A). The circled area B2, which tissue image is similar to the tissue image in FIG. 6A, contrasts to tissue in B1, which is surrounding SRF2, and therefore has been impacted by the sustained release of the cytotoxic agent. This indicates the desired effect of SRF2 on the target tissue over the 30-day period.

FIG. 7 shows systemic drug concentrations measured by LCMS as a function of time post of a SRF1 injection per animal.

FIG. 8 shows systemic drug mean concentrations measured by LCMS (liquid chromatography/mass spectrometry) as a function of time post SRF1 injection per animal as a function of drug dosage. N=6 for 0 to 30 days and N=3 60 days, 4 animals w 5 mg and 2 animals with the 10 mg initiation.

FIG. 9 shows locations for injection of SRF1 in a prostate. It shows the locations in TABLE 4 for collecting prostate samples for pharmacokinetic (pK) tissue analysis. Circles indicate treatment samples ("tx"), rectangles indicate either adjacent reference samples ("adj") or distal reference samples ("distal"). Numbers in diagram are the locations corresponding to TABLE 4.

## DETAILED DESCRIPTION

The technical information set out below may, in some respects, go beyond the wording of the present claims. Such technical information is provided to place the claim scope in a broader technical context and to illustrate possible related technical developments.

For purposes of this disclosure, the following terms and definitions apply:

The following are examples of the polymer naming nomenclature. Other examples not explicitly spelled out here use the same rationale: PLGA8515A (0.3 dl/g) means poly(lactide-co-glycolide) with a monomer ratio of 85/15, end capped with acid groups (A), and an inherent viscosity of 0.3 dl/g; and PLGA6535E (0.5 dl/g) means poly(lactide-co-glycolide) with a monomer ratio of 65/35, end capped with ester groups (E), and an inherent viscosity of 0.5 dl/g; and Poly(lactide-co-glycolide) is typically poly(D,L-lactide-co-glycolide) but could also be e.g. any or a mixture of poly(D,L-lactide-co-glycolide), poly(D-lactide-co-glycolide), and poly(L-lactide-co-glycolide).

The terms "about" or "approximately" is defined herein as 30%, 20%, 15%, 10%, 5%,4%, 3%, 2%, 1.5%, 1%, between 1-2%, 1-3%, 1-5%, or 0.5%-5% less or more than, less than, or more than a stated value, a range or each endpoint of a stated range, or a one-sigma, two-sigma, three-sigma variation from a stated mean or expected value (Gaussian distribution). For example, dl about d2 means dl is 30%, 20%, 15%, 10%, 5%, 4%, 3%, 2%, 1.5%, 1 %, 0% or between 1-2%, 1-3%, 1-5%, or 0.5%-5% different from d2. If dl is a mean value, then d2 is about dl means d2 is within a one-sigma, two-sigma, or three-sigma variance from dl. It is understood that any numerical value, range, or either range endpoint (including, e.g., "approximately none", "about none", "about all", etc.) preceded by the word "about," "substantially" or "approximately" in this disclosure also describes or discloses the same numerical value, range, or either range endpoint not preceded by the word "about," "substantially" or "approximately."

The term "drug" or "agent" as used herein is defined as a therapeutic substance, other than food, used in the prevention, diagnosis, alleviation, treatment, or cure of disease. Unless stated otherwise, "drug" and "agent" shall have the same meaning.

The term "cytostatic" as used herein refers to a drug that is non-toxic to cells but does mitigate cell proliferation and permit cell migration. Cytostatic drugs may include without limitation rapamycin, sirolimus, everolimus, zotarolimus, myolimus, temsirolimus, tacrolimus, macrolide antibiotics, ridaforolimus, biolimus, novolimus, deforolimus, structural derivatives and functional analogues of rapamycin and any macrolide immunosuppressive drug. mTOR/PI3K dual inhibitors may also be utilized including dactolisib, BGT226, SF1126, PKI-587, and NVPBE235, mTORC1/mTORC2 dual inhibitors may also be utilized including sapanisertib, AZD8055, AZD2014 as derived from morpholino pyrazolopyrimidine.

The term "cytotoxic" as used herein refers to a drug that inhibits cell growth and proliferation such as chemotherapeutics. These drugs may include but are not limited to pactlitaxel, taxanes, protaxel, vincristine, etoposide, nocodazole, indirubin, anthracycline derivatives, daunorubicin, daunomycin, plicamycin, tauromustine, bofumustane, and plicamycin. These drugs may also be apoptotic such as TGF, topoisomerase inhibitors, including, 10-hydroxycamptothecin, irinotecan, and doxorubicin.

The term "composition" as used herein means a product of mixing or combining various elements or ingredients.

The term "sustained release formulation (SRF)" as used herein refers to a substance for treating BPH, the substance including a drug (or drugs) and carrier for the drug(s) or drug carrier comprising a polymer composition administered to the target tissue in liquid, gel or solid form using a delivery vehicle, whereupon local delivery to the target tissue the sustained release formulation is effective in producing a sustained release of the drug(s) to a targeted tissue of the prostate, thereby producing an efficacious result over a period of time, e.g., from about 1 to 12 months, or up to 2 years following treatment.

Drug or drug combinations used in the SRF include a cytostatic drug, cytotoxic drug, and/or other drugs. The other drug(s) may be used by themselves (i.e., the "other drug(s)" are the only active agents in the SRF), or in combination with the cytostatic drug or cytotoxic drug as part of the medical procedure for treatment of BPH. For example, the other drug(s) may be administered before a SRF including the cytostatic or cytotoxic drug is administered to the target tissue, included in the delivery vehicle with the SRF, which contains the cytostatic drug or cytotoxic drug administered to the target tissue, or administered after the SRF containing the cytostatic drug or cytotoxic drug is administered to the target tissue.

These other drugs, which may be administered with the cytostatic or cytotoxic drug, include alpha blockers or 5-alpha reductase inhibitors. Alpha blockers may include terazosin, doxazosin, tamsulosin, alfuzosin, and silodosin. 5-alpha reductase inhibitors may include finasteride and dutasteride. Anti-inflammatory drugs may include but are not limited to corticosteroids such as dexamethasone, fluticasone propionate, triamcinolone acetonide, mometasone furoate, prednisone, hydrocortisone, estradiol, clobetasol, and budesonide. Non-steroidal drugs may include acetaminophen, ibuprofen, and naproxen. These other drug types may block cytokine activity or inhibit binding of cytokines to inhibit inflammatory signals such as anti-IL1, anti-IL 2, anti-IL3, anti-IL4, anti-IL8, anti-IL15, anti-IL 18, anti-MCP 1, anti-CCR2, anti-GM-CSF, anti-TNF antibodies and others.

Bioabsorbable refers to the disappearance of a compound into another substance. Biodegradable refers to cell mediated degradation resulting in cleavage of polymer molecular mass and generation of degradation by-products. Bioresorbable includes biodegradation and also total elimination by dissolution, excretion, or assimilation.

The term "target tissue" as used herein is defined as a tissue of the prostate tissue to include the transition zone, peripheral zone and central zone of the prostate, and the prostate. The term "dosage" as used herein is defined is the amount of the sustained release formulation administered to the target tissue using the delivery vehicle, the amount of the one or more drug(s) component(s) of the sustained release formulation, and/or the drug carrier, unless specified otherwise, and in an amount intended to produce a programmed, sustained release and efficacious outcome. This programmed, sustained release and efficacious outcome may be measured using the International Prostate Symptom Score (IPSS), or more generally relieving Lower urinary tract symptoms (LUTS) include voiding or obstructive symptoms such as hesitancy, poor and/or intermittent stream, straining, feeling of incomplete bladder emptying, and storage or irritative symptoms such as frequency, urgency, urge incontinence, and nocturia.

Five alpha reductase inhibitors reduce the prostate volume by 50% when given orally. Minimal reduction occurs in less than six months. An up to 50% reduction in prostate volume is expected in 12-24 months or possibly longer with appropriate therapy.

Alpha blockers can also be used to treat symptomatically at the time of procedure by blocking the alpha receptor and relaxing the prostate smooth muscle. Alpha blockers, five alpha reductase inhibitors or both may be co-formulated with cytostatic or cytotoxic drugs in the SRF.

When expressing a % of a substance in the SRF, the % of that substance may be expressed in terms of a percent weight of the drug(s) to the overall weight of the SRF("% X by wgt"), or to the overall volume of the SRF("% X by vol"). Unless stated otherwise the percent dosage % will, by default, always refer to a % by weight to the total measured SRF. Unless stated otherwise, weights are given in grams ("g") or milligrams ("mg"), molecular weight in kilo-Daltons ("kDa"), volume in microliters ("μL"), and viscosity units are expressed as inherent viscosity (i.e., the ratio of the natural logarithm of the relative viscosity to the mass concentration of the substance, such as a polymer. The unit of inherent viscosity is deciliters per gram (dL/g). A different measure of viscosity is intrinsic viscosity, which is a measure of a solute's contribution to the total viscosity. Another viscosity is dynamic viscosity, the units of which are centimeter-gram-seconds, also known as centipoise (cP).

The drug carrier portion of the SRF generally includes a polymer composition. A solvent, used in the preparation of the SRF, and/or other substances may also be present with the SRF, such as an ultrasound / echoing enhancing medium or other imaging enhancing depending on the imaging modality used.

The SRF may comprise 0.1-60% of a polymer composition, or more preferable 10-50% the polymer composition. The SRF may comprise 0-80% solvent. The drug to polymer weight ratio of the SRF may be 1:100, 1:50, 1:25, 1:20, 1:10, 1:5, 1:2, 1:1, 2:1, or 5:1. The SRF, once located at the target tissue, may release 1-10%, or 11-50% of the drug load in less than 24 hours, 24-72 hours, 3-7 days, 1-4 weeks, 1-3 months or more than 3 months. The SRF may release 80-100% in 24-72h, 3-7 days, 1-4 weeks, 1-3 months or more than 3 months.

The drug carrier may be a polymer composition including silk-elastin like protein polymers, Pluronics F68 or F127 or a combination thereof, poly(ε-caprolactone) (PC), polylactides (PLA), poly(D,L-lactide) (PDLA), poly(ortho esters), polyanhydrides, polycarbonates, polyethylene glycol (PEG), polyethylene oxide (PEO), polyesteramides, and any combinations thereof including block and random co-polymers such as but not limited to poly(lactide-co-glycolide) (PLGA) and PLGA-PEG-PLGA. More specifically the PLGA composition may consist of poly(D,L-lactide-co-glycolide) (50:50), poly(D-lactide-co-glycolide) (50:50), poly(L-lactide-co-glycolide) (50:50), poly(D,L-lactide-co-glycolide) (65:35), poly(D-lactide-co-glycolide) (65:35), poly(L-lactide-co-glycolide) (65:35), poly(D,L-lactide-co-glycolide) (75:25), poly(D-lactide-co-glycolide) (75:25), poly(L-lactide-co-glycolide) (75:25), poly(D,L-lactide-co-glycolide) (85:15) or a mixture thereof. The PLGA may be end-capped with ester, acid, alcohol, thiol or other end-groups. The inherent viscosity of the PLGA polymer may vary from 0.2 dL/g to greater than 1.0 dL/g. The molecular weight of the PLGA polymer may vary from 10 kDa up to 150 kDa. The polymer may be linear, branched, hyperbranched, dendritic, have a star structure, or be a dendrimer-like star polymer.

The drug carrier may include a polymer composition including poly(lactide-co-glycolide), poly(D,L-lactide-co-glycolide), poly(D,L-lactide), ester end capped poly(D,L-lactide-co-glycolide) (50-50), ester end capped poly(D,L-lactide-co-glycolide) (65-35), ester end capped poly(D,L-lactide-co-glycolide (75-25), ester end capped poly(D,L-lactide-co-glycolide (85-15), acid end capped poly(D,L-lactide-co-glycolide) (50-50), acid end capped poly(D,L-lactide-co-glycolide) (65-35),acid end capped poly(D,L-lactide-co-glycolide (75-25), acid end capped poly(D,L-lactide-co-glycolide (85-15), ester end capped poly(D-lactide-co-glycolide) (50-50), ester end capped poly(D-lactide-co-glycolide) (65-35), ester end capped poly(D-lactide-co-glycolide (75-25), acid end capped poly(D-lactide-co-glycolide) (50-50), acid end capped poly(D-lactide-co-glycolide) (65-35),acid end capped poly(D-lactide-co-glycolide (75-25), ester end capped poly(L-lactide-co-glycolide) (50-50), ester end capped poly(L-lactide-co-glycolide) (65-35), ester end capped poly(L-lactide-co-glycolide (75-25), acid end capped poly(L-lactide-co-glycolide) (50-50), acid end capped poly(L-lactide-co-glycolide) (65-35),acid end capped poly(L-lactide-co-glycolide (75-25), ester end capped poly(D,L-lactide-co-glycolide), acid end capped poly(D,L-lactide-co-glycolide), or combinations thereof.

The drug carrier may include a bioabsorbable polymer and the inherent viscosity of the polymer is between 0.1 - 1.0 dL/g, 0.1 - 0.6 dL/g, or 0.1 to 0.4 dL/g or 0.1 to 0.3 dL/g and the ratio of DL-lactide to glycolide is from 30/70 up to 90/10, 95/5, or 85/15.

The composition may include a bioabsorbable polymer at a concentration of 20 - 80%, 25 - 75%, 40 - 60% by wt. of the bioabsorbable polymer, 80-20%, 75-25%, 60-40%, by wt. of the solvent and 0.5% - 30% by wt. drug; 1% - 20% by wt. of drug, or 1% - 5% by wt. of drug.

The drug carrier may generally be in the form of amorphous or semicrystalline, homogenous, or phase-separated, and provided in the form of a liquid solution or, suspension, or as nanoparticles, microspheres or microparticles processed by spray drying, emulsion, electrospray, or extrusion. The biodegradable polymer is preferably chosen to substantially biodegrade in a period of about 3 to 6 months or 6 to 12 months.

In some embodiments it may be desirable to formulate the SRF so that the drug carrier is fully biodegraded before the next treatment, e.g., 3 months, 6 months or 12 months after the prior treatment. For example, the polymer would have a ratio of glycolide to lactide of 70:30 up to 15:85 for a more hydrophilic property (faster degradation) and /or an inherent viscosity less than about 1.0 dL/g for a more hydrophilic property.

A polymer composition, when forming a constituent of the SRF, is a polymer composition that enables or achieves a desired "sustained release" of the one or more drugs to the target tissue. In some embodiments, the polymer composition enables or achieves at least 50%, or up to about 100%, or substantially all drug release between 30 and 90 days, through a combination of diffusion and degradation. In other embodiments up 100% of drug release occurs from 90 to 120 days from treatment. Preferably, there is an initial burst (e.g., up to 50% of drug) followed by a substantially reduced rate of release over the next following month, or several following months following treatment. For example, the drug has a release rate of between 5% to 50% during the first 24 hours from injecting the composition into the prostate and the drug has a release rate of no more than 10% to 75% over the first month, 25% to 95% over the first three months, and/or 50% to 100% over the first six months.

A programmed, sustained release of, e.g., from 14 days or 1 to about 12 months for substantially all of the drug to dissipate from the carrier is achieved by selection of drug carrier (polymer structure) and/or modifying the morphology and mechanical properties (stiffness of the polymer), the polymer/drug ratio, controlling the physical shape/dimensions (volume) of the SRF and/or composition that is delivered to the target tissue. Other factors affecting the release rate include:

ability of polymer to swell (controlled by e.g. monomers selection and monomer ratios = polymer structure).

porosity/morphology (controlled by e.g. polymer structure and concentration, polymer/solvent ratio and miscibility, and polymer/drug ratio- less drug than polymer than drug is trapped).

how fast the materials gel and whether glass transition is reached - transition from liquid to solid using water soluble or insoluble solvents (fast gelation leads to faster initial release / burst) (controlled by e.g. polymer structure and concentration, polymer/solvent ratio and miscibility).

drug/polymer miscibility and polarity of solvent, and

molecular weight and lipophilicity of drug.

The SRF has to keep drug exposure near the prostate and minimize leak or flow to other surrounding organs. In addition, the SRF should not take up too much volume in the prostate. A desired shape of the drug release curve could be a burst of drug and early tissue exposure for fast efficacy and then reduced rate of drug release over a 3-6 month period for sustained efficacy.

For example, a fast burst release rate followed by a reduced rate was achieved with N-methyl pyrrolidone (NMP) solvent - water soluble. This formulation can provide a high release within the first 24 or 48 hours, or within the first 1 week, two weeks, or up to 3 months, followed by a reduced rate of release.

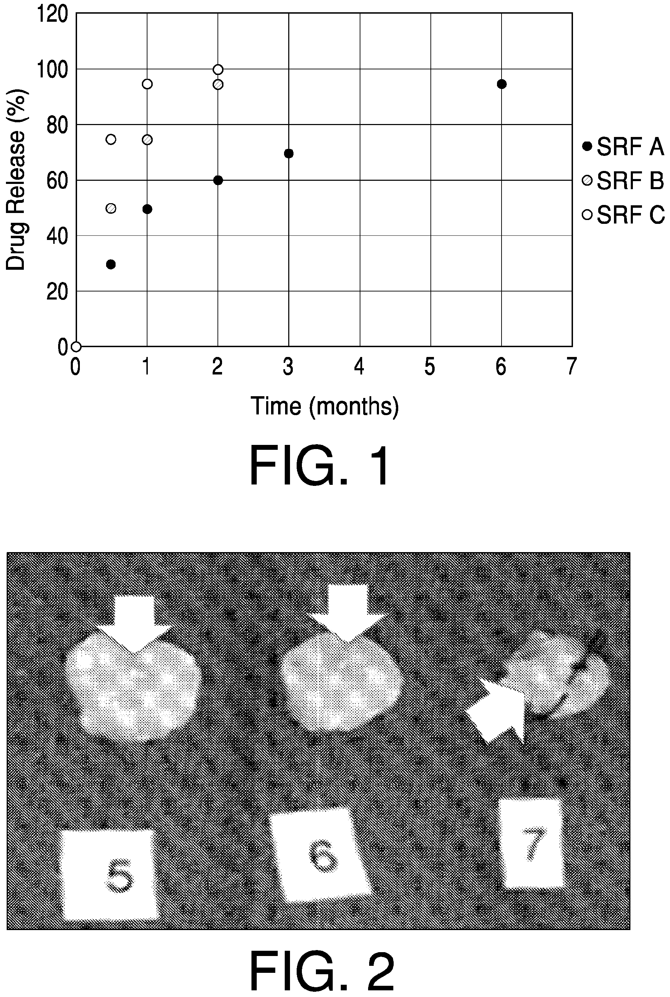

FIG. 1 shows a Drug Release Curve for Sustained Release Formulation (SRFs) A, B and C. These exemplary SRFs provide a burst followed by gradual release of drug over a period of up to 6 months. SRF A releases about 25% of the drug within the first month, followed by a slow, gradual release where about 95% is released at six months. SRF B has an initial burst of about 50% within the first month, followed by about 100% release at two months. SRF C has an initial burst of about 75% within the first month, with about 100% release at about two months.

TABLE 1A below shows examples of SRF formulations for each of SRF A, B and C. Examples A1, A2 exhibit approximately the same release rate characteristics as SRF A in FIG. 1. Examples B1, B2 exhibit approximately the same release rate characteristics as SRF B in FIG. 1. Examples C1, C2 exhibit approximately the same release rate characteristics as SRF C in FIG. 1. TABLE 1B shows examples of drug released over time.

| TABLE 1A: Species of SRF formulations for SRF A, B and C in FIG. 1 | | | | |
| --- | --- | --- | --- | --- |
| SRF | polymer | drug | % vol. drug v. polymer | Solvent |
| A1 | PLGA8515 | Sirolimus | 1-2% drug, 50% polymer | NMP |
| A2 | PLGA8515 | Pactlitaxel | 1-2% drug, 50% polymer | NMP |
| B1 | PLGA7525 | Sirolimus | 3-5% drug, 45-47% polymer | NMP |
| B2 | PLGA7525 | Paclitaxel | 3-5% drug, 45-47% polymer | NMP |
| C1 | PLGA5050 | Sirolimus | 1-5% drug, 45-55% polymer | NMP |
| C2 | PLGA5050 | Paclitaxel | 1-5% drug, 45-55% polymer | NMP |

| TABLE 1B plotted ranges for SRF A, B & C (FIG. 1) | | | |
| --- | --- | --- | --- |
| Time (months) | SRF A (drug release %) | SRF B (drug release %) | SRF C (drug release %) |
| 0 | 0 | 0 | 0 |
| 0.5 | 30 ± 20 | 50 ± 25 | 75 ± 25 |
| 1 | 50 ± 20 | 75 ± 15 | 95 ± 5 |
| 2 | 60 ± 15 | 95 ± 5 | 100 |
| 3 | 70 ± 10 | 100 | |
| 6 | 95 ± 5 | | |

The SRF may be delivered to the target tissue in the form of a composition in liquid form (i.e., the SRF's drug(s) and drug carrier are in solution, or in suspension in a solvent when in the delivery vehicle) or as a composition in gel form, either upon contact with water at the target tissue, or as formulated and contained within the needle or catheter. The later examples of a composition including the SRF may be made by dissolving the SRF in a suitable solvent. Suitable solvents for these embodiments include water, N-methylpyrrolidone (NMP), dimethyl sulfoxide (DMSO), 2-pyrrolidone, propylene carbonate, caprolactam, triacetin, alcohols, benzyl benzoate, ethyl acetate, triethyl citrate, benzyl alcohol, glyme (dimethoxyethane), diglyme, and other glycol ethers, and dichloromethane, or any mixture thereof.

The composition should be formulated at a concentration and viscosity permitting passage in a 16G or larger inner diameter needle applicator. Drug to polymer ratios may vary from 0.05 to 2.0. Drug and polymer concentrations in solvent may range from 0.1 wt% up to 60 wt%. Injection volumes of the drug portion of the SRF may range from 25 microliters up to 5 mL per injection. Overall dosage of drug provided in the SRF can range from 50 ug up to 200 mg. For example, a composition containing the SRF is 300 ug of everolimus or paclitaxel dissolved with 300 ug ("ug" - micrograms) poly (D, L-lactide-co-glycolide) (85:15) in NMP at a 50 wt% concentration.

The gel-like properties of some compositions may be beneficial to prevent infection from the rectal area along the needle tract. A sufficiently high viscosity can keep the drug in place, mitigating the possibility that the drug travels through the insertion pathway from target tissue to entry point. In this sense the gel-like composition acts as a sealant.

A gel-like state for the composition (as delivered or after delivery) is believed advantageous because once delivered to the target tissue, the SRF will tend not to dissipate or diffuse to other non-intended treatment areas of the prostate of adjacent tissue. As such, the dosage may be lower when the SRF exists in a gel-like form at the target tissue. More generally, it may be desirable to formulate the SRF to exhibit a high viscosity (e.g., gel-like) but not so high as to impair the ability for a physician to deliver the SRF to the target tissue for similar reasons.

Alternatively, the composition may be adapted to foam when located at the target tissue.

The sustained release formulation may alternatively be provided in the form of rods provided by melt or solvent casting, fiber spinning, electrospinning, injection molding or extrusion. For extrusion the drug of the SRF must be stable at the extrusion temperature necessary to melt the polymer. For example, paclitaxel (melting point approximately 216 °C ) may be mixed at 60 wt% in poly(D,L-lactide-co-glycolide) (50:50) and extruded using a twin screw extruder at less than 200°C above the glass transition at which the amorphous polymer flows through a die to form rods then cut to length. Alternatively, everolimus (melting point approximately 100°C) may be mixed at 60 wt% in polycaprolactone and extruded using a twin screw extruder at less than 100°C or higher temperature above glass transition at which the polymer flows through a die to form rods then cut to length. To enhance the mixing, the drug and drug carrier may be premixed by methods such as spray drying, emulsion, electrospray, extrusion, solvent casting, melt casting. Alternatively, the polymer resin granules and drug powder may be mixed. Particle size reduction may be feasible by known methods such as milling, jet milling, spray drying, electrospray, emulsion, and/or cryogenic methods.

The following disclosure provides examples including non-limiting embodiments for treating prostatic hyperplasia tissues within a patient. Local drug delivery is in the form of the sustained release formulation providing sustained delivery of a cytostatic or cytotoxic drug delivered using a delivery vehicle to the patient. The delivery vehicle may be in the form of a needle or catheter administered by a health professional.

The local drug delivery device applicator may be a needle or catheter for delivery of the composition including the SRF to the target tissue. An applicator portion of the delivery vehicle may include a chamber for holding the drug and drug carrier separate from each other, a mixing element for combining the drug and drug carrier such as a static mixing Y-adapter. Alternatively, the drug and drug carrier may be mixed using two syringes connected with an adapter, with back and forth plunging to mix the drug and drug carrier. The applicator needle or catheter, and/or other component may be imaged under ultrasound to visualize the therapy. Echogenicity may be enhanced by dimpling of the applicator needle or coatings. Echogenicity may also be enhanced by incorporation of contrast enhancing agents such as microbubbles, sulfur hexafluoride, octofluoropropane, air, lipid and/or albumin shells. The applicator may have components for attachment to a rectal ultrasound probe. The applicator may have markings demonstrating measurement of lengths of needle insertion. The applicator may have a user-friendly handle and plunger to deliver the needle or catheter to the target tissue. An applicator needle may range from 16G up to 25G. The needle length may be 40 cm or less (e.g., 20 cm length) in order to reach the target tissue from a transrectal or transperineal approach.

In one example, injection volume of a SRF injectate is minimized between 10-200 microliter per injection over 1-10 injections across each side of the prostate to reduce prostatic tissue pressure on the urethra, which would mitigate any potential backflow through the delivery device and/or loss of therapeutic injectate from the prostate. A further injection volume range 50-100 microliter per injection over 1-5 injections across each side of the prostate was studied. In addition, this injectate volume was found to minimize swelling and bulking of the prostate. Too large of an injectate volume can cause undesirable bulking of the prostate.

Precise delivery of low volumes of a SRF for therapeutic retention with a precise drug dosage may be accomplished using a novel delivery device, such as 250-500 microliter or smaller. This delivery device may have a gastight, glass or polypropylene syringe body with Teflon or polypropylene plunger connected with a 20cm long 22g needle. The volume of the delivery syringe must be large enough to accommodate the dead volume within the needle of approximately 200 microliter. Therefore, a 250 microliter gastight syringe can be utilized to deliver a single 50 microliter SRF1 injection. For multiple injections from the same delivery device a 250-2,000 microliter gastight syringe volume may be used, which allows sufficient volume to overcome needle dead volume and can deliver at least 100 microliter injections to each lobe of a prostate.

A desired injection volume dosing control and precision custom elongated glass body gastight delivery syringes can be utilized that provide increase accuracy of volume graduation markings on the syringe. Instead of volume graduations these delivery device markings can also show directly the needle calibrated dead volume and drug delivery drug dosage in mg for each injection for the user.

An SRF can be provided pre-mixed in solution in the delivery device for each injection resulting in delivery of primarily amorphous drug dissolved in the delivery SRF for enhanced onset of efficacy.

An SRF can be provided pre-mixed in solution in a delivery device for each therapy (number of pre-filled devices = total # of injections for each therapy) resulting in delivery of primarily amorphous drug dissolved in the delivery SRF for enhanced onset of efficacy.

An SRF can be provided with polymer injectate and drug powder in separate delivery syringes that are mixed by the user on or about the time of treatment. This would allow for a crystalline drug directly into the injectate to increase drug retention time and exposure and reduce systemic drug loss.

An SRF may be mixed with an ultrasound microbubble contrast agent to further enhance visibility of injectate under transrectal ultrasound guidance to enable precise location control of injection within a prostate capsule within each lobe to further minimize potential for drug loss to the urethra, surrounding organs and systemic circulation.

Drug morphology can include some combination of amorphous and crystalline drug to provide sufficient immediate drug release for onset of efficacy and to extend local drug exposure time and reduce systemic washout

Drug properties are lipophilic or hydrophobic to extend local drug exposure time and reduce systemic washout compared to a hydrophilic drug, peptide or protein that is more readily bioavailable systemically.

Drug to polymer ratio is an important factor. Too low of a drug to polymer ratio is not attractive given if the drug dosage is too low then it results in the local drug concentrations being too low. Too high of a drug to polymer ratio results in the drug releasing from the SRF too fast which limits the longer term local drug release and also leads to systemic drug exposure. An optimal drug to polymer ratio Is 5% to 30% by weight. More specifically it may be preferred to utilize 7.5% to 12.5% by weight for optimal drug release and retention.

An SRF can be used before, together with or after other prostate transurethral procedures such as Rezum, Urolift, TURP or others to reduce further prostate enlargement, scar formation and inflammation post-procedure and reduce need for a repeat procedure

A SRF platform can be utilized in treatment of any localized hyperplastic tissue abnormality such as vascular restenosis, localized tumors such as prostate cancer, etc.

The various advantages of the invention described herein may be, as mentioned earlier, practiced in the form of stand-alone treatment or in combination with a known treatment for BPH administered before, during or after such treatment. Those known treatments for BPH may include balloon dilation, stenting, transurethral incision, transurethral resection (TURP), transurethral needle ablation, transurethral microwave therapy, electrical vaporization, water vapor thermal therapy, prostatic urethral lift (PUL) implants, etc.

The known procedures have demonstrated varying levels of efficacy, as well as undesirable or adverse indications, complications associated with the invasive aspects of the treatment, and/or negative patient experiences. TURP produces improved efficacy and improvement in urinary flow rate and symptom score (IPSS) but is invasive with significant side effects on incontinence, urgency, dysuria, acute retention, stricture, ejaculation dysfunction and sexual dysfunction. Water vapor therapy and PUL have demonstrated less sexual dysfunction side effects but are limited to use in smaller BPH prostates less than 80 ml and have shown less efficacy with non-responders and higher retreatment rates compared to TURP. Furthermore, all procedures are invasive and require transurethral access and catheter placement.

Less invasive targeted drug delivery approaches to the prostate zone have been attempted by the transrectal or transperineal routes such as pore forming proteins and peptides in saline formulations with single dosages but demonstrated limited efficacy versus saline placebo in randomized clinical trials. See Indian J Urol. 2008 Jul-Sep; 24(3): 329-335. doi: 10.4103/0970-1591.42613, PMCID: PMC2684358, PMID: 19468462; Injection therapy for prostatic disease: A renaissance concept. Arash M. Saemi, Jeffrey B. Folsom, and Mark K. Plante. Additionally, alcohol or medications injected into the prostate have been ineffective. Alcohol single injection is very caustic and poorly controls the area of delivery. Medication injection into the prostate is also ineffective as it is given in a single dose with poor effect. Other attempts to treat prostate using similar drugs have been used, such as drugs taken orally, or if injected, the injectate did not include a sustained release formulation of the drug and thus an efficacious response in the target tissue injected would not be exhibited.

A delivery of the SRF to the target tissue as described, may also be used with, prior to, or after delivery of a drug eluting implant or stent designed to maintain patency of the urethra. This implant may be composed of nitinol or bioabsorbable polymers such as PLGA. The implant may be delivered by a transurethral, transrectal or transperineal approach. The implant may consist of a shape set nitinol wire or extruded polymer fiber that is then coated with drug in a sustained release formulation by dip, air assisted, ultrasonic or electrospray coating. The design may be such that the implant corkscrews around the urethra transition zone and does not impact the lumen of the urethra to minimize adverse effects and side effects.

The SRF may also be used in a complementary manner with an implantable energy generator to deliver direct energy in a continuous or pulsed manner by activation of external stimuli. The energy generator may relieve lower urinary tract symptoms by neuromodulation of the target tissue.

EXAMPLE 1

A preclinical study was conducted to evaluate the safety and feasibility of a composition comprising a sustained release formulation (hereinafter "SRF2") injected into the prostates of two canine models, each having normal (non-enlarged) prostates. 0 and 28 days following the procedure the models ("animal #20C0081", the acute model/animal) were humanely euthanized and evaluated to determine whether there were any acute, toxic effects of the injectate. The day to day behavior of the second model ("animal #20C0083", chronic model / animal) was being studied over the 28-day period following the procedure.

Data collected from each of the models (i.e., the acute and 28-day study):

•    Evaluate Prostate Morphology and Measure Prostate Size/Weight Baseline and Post-Treatment (Ultrasound)

•    Urinalysis, Volume Output and Residual at baseline, post treatment, daily to Termination

•    Gross Necropsy and Camera/Microscope Imaging at Termination, Fixation

•    Histology at Treatment Site at Termination (H&E)

SRF2 is a composition including a sustained release formulation prepared by adding 0.5 mL N-methyl pyrrolidone (NMP) to a vial with 0.25 g paclitaxel and vortexed until dissolved and then taking 100 microliter of that drug solution and adding to 2.5 mL of a 50/50 PLGA8515 NMP solution using syringe to syringe mixing with a female to female luer connector. 100 microliters of SRF2 was loaded in a 1mL syringe with a 20 G x 20 cm Chiba biopsy needle.

Transrectal or transperineal ultrasonography. Transrectal or transperineal prostate block with local anesthetic with 20 gauge syringe. Insertion of drug via the same 20 gauge needle into each lobe of the prostate. Positioning of the implanted medication confirmed by ultrasound. Removal of the ultrasound probe and needle.

Transrectal ultrasound was used to evaluate the size and condition of the prostate before and after treatment. Each animal received three injections of SRF2 (total dosage is 300 microliters) in the prostate under ultrasound guidance.

Animal #20C0081

At the end of the procedure, animal #20C0081 was humanely euthanized and sent to necropsy. For animal #20C0081 the needle guide and biopsy needle were attached to the ultrasound rectal probe. The probe was advanced to the first prostate lobe. Prostate volume was measured to be 3.08 cm^3 via the equation:

$$\text{Prostate volume} = 0.5233 \text{ x TRD x APD x LD}$$

Where TRD was 1.83 cm, APD was 1.19 cm and LD was 2.70 cm.
(transverse diameter (TRD) anteroposterior diameter (APD), longitudinal diameter (LD))

Figure 1 of RTPROV-2 shows the transrectal ultrasound volume measurement of the canine prostate in animal #20C0081. 100 microliter of SRF2 was injected into the left prostate lobe as shown in Figure 2 of RTPROV-2. The probe was advanced to the second prostate lobe. 100 microliters of SRF2 was injected into the right prostate lobe. A second 100 microliter of SRF2 was injected into the right prostate lobe after the first injection was outside the lobe. The 200 microliters of SRF2 injectate can be visually seen as less than 10% of prostate volume under ultrasound.

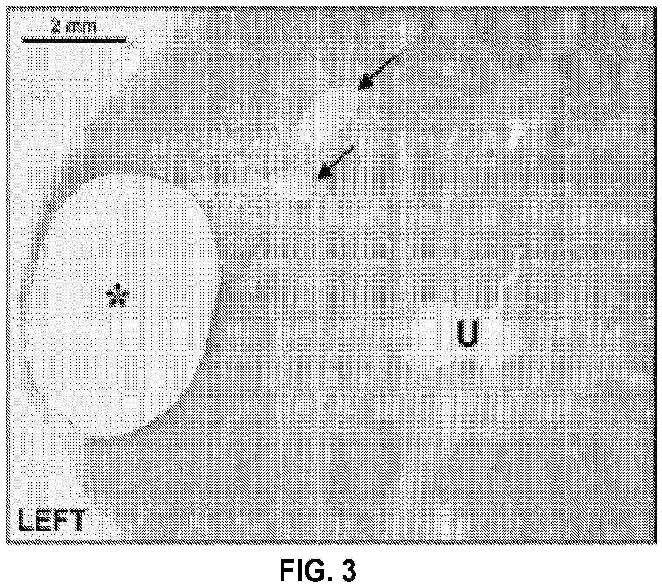

The successful treatment is shown in Figure 3 of RTPROV-2.

Figure 2 of RTPROV-2 is an image showing treatment (injecting SRF2 into the prostate) that used a 20G x 20cm Chiba biopsy needle.

Figure 3 of RTPROV-2 is an image showing the SRF2 injectable as visible in canine prostate.

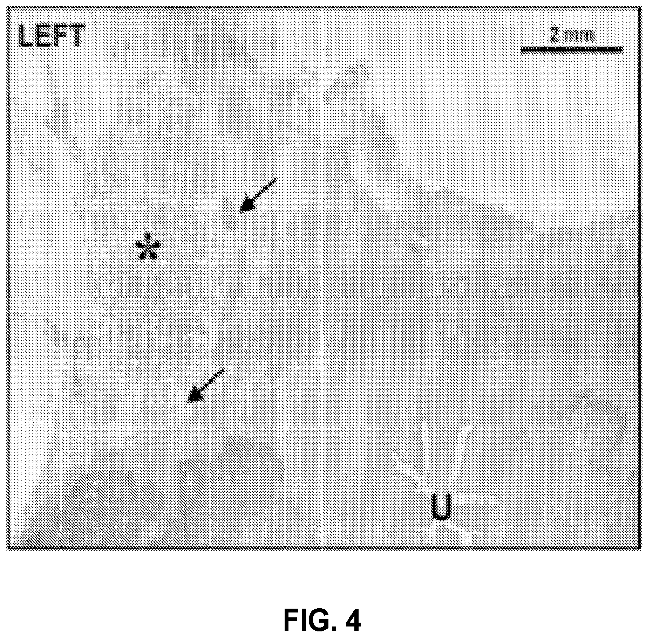

After treatment with SRF2. The animal was humanely euthanized, and the prostate and surrounding bladder and urethra explanted. The SRF2 injections visibly observed in the explanted prostate, as shown in Figure 4 of RTPROV-2.

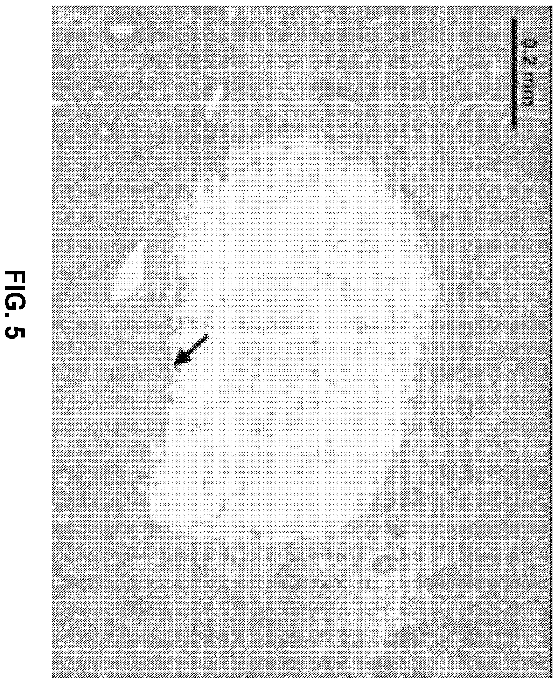

Figure 5 of RTPROV-2 indicates no significant adverse effects were observed due to the procedure or injectate.

Animal #20C0083

For animal #20C0083 (chronic model/animal), the needle guide and 20G x 20cm Chiba biopsy needle was attached to the ultrasound rectal probe. The probe was advanced to the first prostate lobe. Prostate volume was measured to be 3.52 cm^3 via the equation:

$$\text{Prostate volume} = 0.5233 \times TRD \times APD \times LD$$

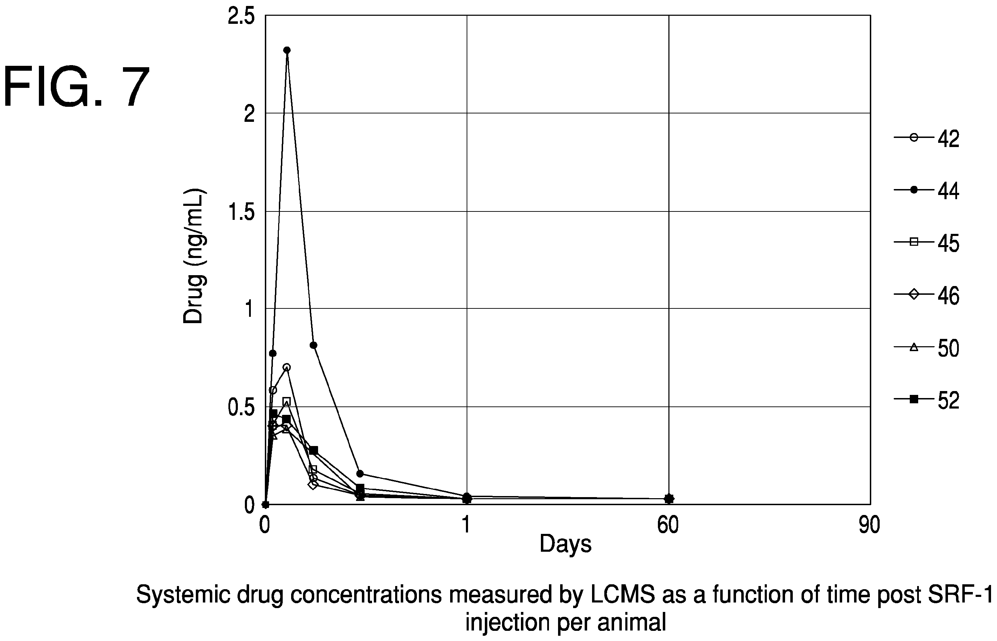

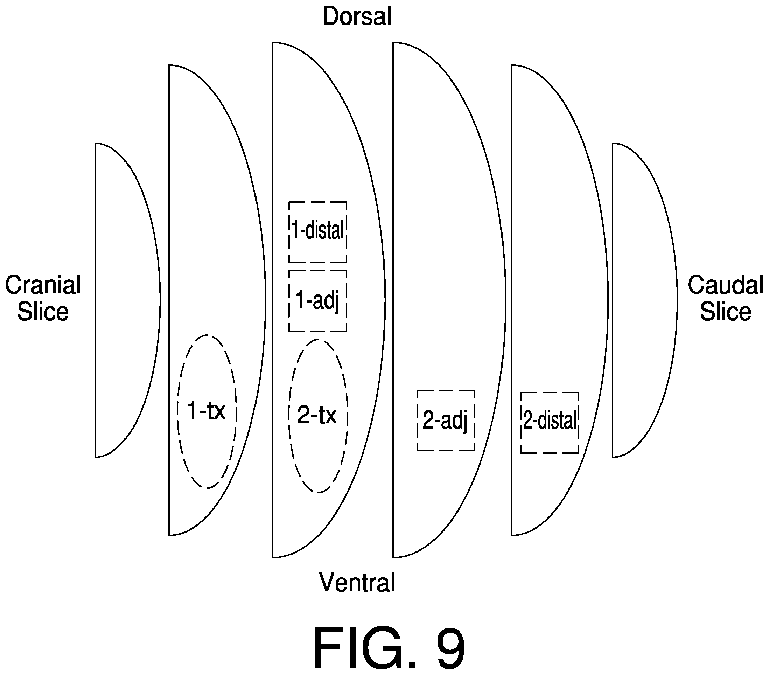

Where TRD was 1.88cm, APD was 1.32cm and LD was 2.71 cm (Figure 6 of RTPROV_2).
100 microliter of SRF2 was injected into the left prostate lobe. The probe was advanced to the second prostate lobe. 100 microliters of SRF2 was injected into the right prostate lobe. A second injection of 100 microliter of SRF2 was injected into the left prostate lobe after the first injection was outside the lobe. Prostate SRF2 injections were retained and visible in the prostate as observed under ultrasound (Figures 7-9 of RTPROV-2). The animal recovered normally from anesthesia. The animal appeared bright, alert and comfortable, with slightly nervous temperament. The animal also had normal urine present in the pan liner the morning following the procedure.

Observations from study

FIG. 2 shows gross pathology images of trimmed and formalin fixed treated prostate gland from a canine study acute animal 20C0083. Visible white material demonstrates successful, localized injection of an SRF. These areas are identified by the white arrows in FIG. 2. The delivery vehicle (needle injection, the needle containing SRF2) is described above. The SRF localized to the areas shown indicates that the SRF had not undergone any significant diffusion into adjoining tissue, which is desired. The SRF was localized to the area where the injectate was placed.
FIG. 3 shows an H&E (hematoxylin and eosin) stained histopathology image of a treated prostate from the acute animal 20C0083 with non-staining injectate material (identifying the presence of the SRF) in the left lateral lobe (asterisk). (U = urethra, arrows designate needle tracks). This image further indicates that the injectate, containing the SRF (SRF2), was localized without any significant diffusion into adjoining tissue for the acute animal. It is believed that without the SRF formulation of the drug and polymer there would have been significant diffusion of the cytotoxic drug, which is undesirable for at least two reasons. First, to treat the area effectively a higher wt% of drug injectate may be needed since the concentration of drug at the target tissue is reduced due to diffusion. Second, the drug, by diffusing to other areas may introduce adverse consequences. Hence it is desirable to minimize the amount of diffusion so that only the target tissue receives an effective amount of drug to treat the tissue.
FIG. 4 shows a chronic 30-day animal 20C0081 histopathology H&E stained image demonstrating degenerate smooth muscle in the left sided cranial prostate subsample (arrows). (U = urethra). It is believed that a key pathology of BPH is the proliferation of smooth muscle cell. The drug portion of the SRF is intended to degenerate smooth muscle cells or prevent their proliferation. The image shows that SRF2 was effective over a 30-day period in breaking down smooth muscle cells.
FIG. 5 shows the chronic animal 20C0081 histopathology H&E stained image demonstrating prostate tissue surrounding injectate and loss of adjacent glandular acini adjacent (arrow). The image indicating local loss of the glandular tissue evidence both drug effectiveness and desired treatment only at the target tissue.
FIGS. 6A and 6B are comparison images that further evidence the effect of SRF after 30 days and its localized treatment, or lack of diffusion of the drug to tissue adjoining the target tissue or tissue nearby. FIG. 6A shows the target tissue morphology of the acute animal 20C0083 (histopathology H&E stained). FIG. 6B shows the target tissue of the chronic animal 20C0081 (Masson's trichrome stained). The white area in the image is the location of the target tissue where the SRF was injected. Comparing FIGS. 6A to 6B, there is a loss of the glandular acini shown in the acute animal (FIG. 6A). The circled area B2, which tissue image is similar to the tissue image in FIG. 6A, contrasts to the tissue identified in B1, which is surrounding the SRF. The SRF has impacted the tissue in the region of B1 by a sustained release of the cytotoxic agent. This image indicates that the desired effect of the SRF on the target tissue over the 30-day period has taken place. Additionally, this image indicates the lack of drug diffusion to nearby tissue, which is also desirable.

EXAMPLE 2

A preclinical study was conducted to evaluate the safety and feasibility of a composition comprising an SRF (SRF1) injected into the prostates of six canine models, each having enlarged prostates post weekly treatments by testosterone injection over a period of twelve weeks. The SRF1 treatment was delivered 12 weeks post the initial testosterone injections. The composition comprising SRF1 was delivered to these BPH canine models in the following manner:

SRF1 included 5mg paclitaxel delivered to a BPH canine model prostate (N=4) in 100 microliter SRF1 injectate by injecting 50 microliter injections into each side of the prostate by transrectal ultrasound guidance using a 20G x 20cm Chiba needle delivered via 250 microliter gastight glass delivery syringe. The injectate was easy for the user to deliver and visualize under ultrasound without discomfort to the animal. A total of 2 injections were made to each BPH canine model to deliver SRF1.

10mg paclitaxel was delivered to the BPH canine model prostate (N=2) in 200 microliter SRF1 injectate by injecting 100uL injections into each side of the prostate by transrectal ultrasound guidance using a 20G x 20cm Chiba needle delivered via 500 microliter gastight glass delivery syringe. The injectate was easy for the user to deliver and visualize under ultrasound and did not cause any discomfort to the animal. A total of 2 injections were made to each BPH canine model to deliver SRF1.

Blood samples were collected from all animals at 0, 1, 3, 7, 14, 30, 60 and 90 days post treatment or until the animal was euthanized. 30 and 90 days following the procedure the models were humanely euthanized and evaluated to determine whether there were any acute, toxic effects of the injectate. At the time the animals were euthanized tissue samples were also collected from the prostate, bladder, and urethra to determine the residual drug concentrations. The day-to-day behavior of all animals was also studied over the 30-and-90 days periods following the procedure.

Data collected from each of the models (i.e., the 30- and 90-day studies):

Prostate Morphology and Prostate Size/Weight Baseline and Post-Treatment (Ultrasound)

Gross Necropsy and Camera/Microscope Imaging at Termination, Fixation

Histology at Treatment Site at Termination (H&E)

TABLE 2 shows prostate volume (cc) as measured by ultrasound at baseline (i.e., before the animal received SRF1) and 4 weeks and 12 weeks post SRF1 treatment. TABLE 2 shows a reduction in prostate volume (%) post SRF1 treatment relative to baseline.

| TABLE 2 | | | | | | |
|---|---|---|---|---|---|---|
| | | Prostate Volume by Ultrasound (cc) | | | Reduction from Baseline in Prostate Volume (%) | |
| Animal# | Dose (mg) | Baseline | 4 weeks post SRF1 | 12 weeks post SRF1 | 4 weeks post SRF1 | 12 weeks post SRF1 |
| 50 | 5 | 39.2 | 10.6 | | -72.9 | |
| 45 | 5 | 26.4 | 7.53 | | -71.4 | |
| 42 | 10 | 29.7 | 5.87 | | -80.2 | |
| 46 | 5 | 19.5 | | 13.7 | | -29.9 |
| 52 | 5 | 22.2 | | 8.65 | | -61.0 |
| 44 | 10 | 44.1 | | 8.76 | | -80.1 |

The BPH canine models in the study are distinguished by number. Animal models 45, 46, 50, and 52 received the 5mg drug dose of SRF1. BPH canine models 42 and 44 received the 10 mg drug dose.

| TABLE 3 | | | | |
|---|---|---|---|---|
| | Prostate Volume by Ultrasound (cc, Mean) | | % Prostate Volume Reduction (Mean) | |
| Dose (mg) | 4 weeks post initial testosterone injections | 12 weeks post initial testosterone injections | 4 weeks post SRF1 | 12 weeks post SRF1 |
| 5 | 9.1 | 11.2 | -72.2 | -45.5 |
| 10 | 5.9 | 8.8 | -80.2 | -80.1 |

TABLE 3 shows the mean average change in canine prostate volume (cc) as a function of the drug dose injected (5mg and 10mg) and measured by transrectal ultrasound, post testosterone injections (4 and 12 weeks) and post SRF1 treatments (4 and 12 weeks).

| TABLE 4 | | | | | | |
|---|---|---|---|---|---|---|
| | Overall | | 5mg dose | | 10mg dose | |
| | 30-day (ug/g) | | 30-day (ug/g) | | 30-day (ug/g) | |
| Target Tissue | Mean | Standard Deviation | Mean | Standard Deviation | Mean | Standard Deviation |
| Main Bladder | 0.10 | 0.14 | 0.10 | 0.12 | 0.29 | |
| Bladder neck | 0.05 | 0.06 | 0.02 | 0.02 | 0.12 | |
| Adventia / Adipose Tissue | 1.20 | 2.05 | 1.80 | 2.51 | 0.02 | |
| Prostate tx1 | 16.01 | 6.91 | 20.00 | 0.00 | 8.03 | |
| Prostate tx2 | 17.07 | 5.08 | 20.00 | 0.00 | 11.20 | |
| Prostate adj1 | 13.92 | 9.59 | 19.45 | 0.78 | 2.87 | |
| Prostate adj2 | 11.15 | 7.37 | 9.17 | 9.23 | 15.10 | |
| Prostate distal 1 | 6.23 | 9.17 | 9.12 | 10.87 | 0.46 | |
| Prostate distal 2 | 1.46 | 1.75 | 2.17 | 1.76 | 0.04 | |
| Prostate remains | 9.28 | 10.03 | 13.86 | 8.68 | 0.12 | |
| Urethra proximal | 1.10 | 1.82 | 1.60 | 2.26 | 0.10 | |
| Urethra distal | 0.06 | 0.05 | 0.03 | 0.03 | 0.11 | |
| | | | | | | |

TABLE 4 shows the overall (N=3) mean and standard deviation of drug concentrations (ug/g) in the various organ and tissue 30 days post SRF1 treatment. TABLE 4 also shows the mean (and standard deviation for 5 mg) of drug concentrations (ug/g) in the various organ and tissue 30 days post SRF1 treatment for the two doses delivered, 5 mg (N=2) and 10 mg (N=1).

It is known that for aging men with a history of BPH and a reduced serum testosterone concentration the size of the prostate is not reduced when serum total testosterone concentration is reduced. See Xia, B.-W. et al, Relationship between serum total testosterone and prostate volume in aging men, Scientific Reports, 11, 14122 (2021). This suggests that the significant prostate volume decrease observed in this study, which potentially could be partially due to reduced testosterone concentrations, was more likely due to the SRF1 injections, and not because of a reduction in, or discontinuation of the testosterone treatments in the BPH canine models.

It will be appreciated by a person of ordinary skill, in view of the teachings in this disclosure and the observations from the study, that there is a capacity for effective treatment, localized to the treatment of BPH, and by a needle injection of a composition including a SRF at the target tissue, in contrast to other methods. While those other methods may show efficacy in reducing BPH, they either may require a more invasive procedure (vs. localized treatment using the delivery device as disclosed herein, such as the needle used in the animal study), more frequent treatment due to diffusion or more generalized treatment of BPH raising the possibility of adverse effects because a higher dosage is needed to treat the area while accounting for leakage or diffusion of the drug to other areas. Adverse effects may include diminished urinary or sexual function. It is desired to have an effective treatment targeting only the target tissue and nowhere else (e.g., avoiding the urethra) and to perform the procedure in a less invasive manner for patient acceptance. In contrast to other methods, a needle injection of the SRF at the target tissue, without significant diffusion, demonstrates a capacity to meet these objectives.

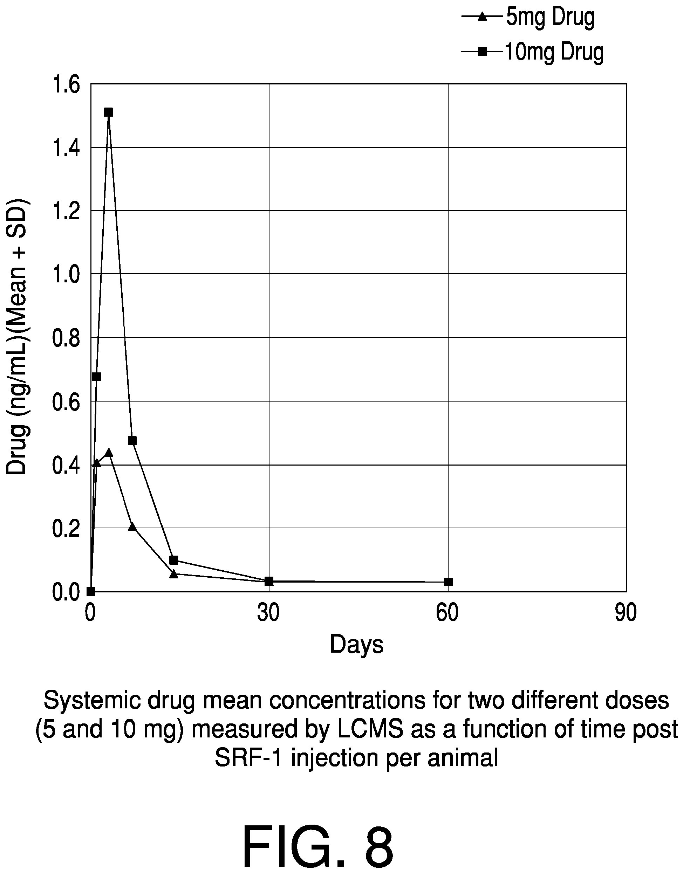

Example 2 describes a study where two injection volumes were considered for BPH canine models. Both injection volumes showed significant reduction in prostate size, as reported in TABLES 2 and 3. Additionally, as shown in FIGS. 7 and 8, there was very low systemic drug concentrations present in the blood, indicating that the agent was contained at the target tissue. Example 2 therefore indicates that an SRF injectate according to the disclosure can (1) reduce prostate volume over a 30 and 90 day period, and (2) limits substantially all of the active agent to the target tissue. As for point (2), it should be mentioned that other known products for treating BPH by contrast have their active agents disperse significantly (systemic drug concentrations much higher than what is shown in FIGS. 7-8). Moreover, the study indicated unexpectedly a significant ratio of prostate 30-day drug concentration to maximum plasma drug concentration of at least about 10,000. As mentioned throughout, it is important to control diffusion of the agent as this can cause adverse effects on nearby tissue and organs. Additionally, in contrast to most prior or existing techniques for treating BPH, a comparatively small volume of SRF injectate is needed for efficacy. As mentioned above, injection volume of a SRF (SRF1) injectate is minimized to between 10-200 microliter per injection over 1-10 injections across each side of the prostate, or 50-100 microliter per injection over 1-5 injections across each side of the prostate. Prior approaches for treating BPH seek to maximize injection volumes for increased drug dosage. An SRF however does not require as high a drug dosage, because the drug is maintained in the target tissue.

Additional considerations include the drug to polymer ratio aspect of an SRF. An appropriate drug to polymer ratio, consistent with the objectives set forth herein, was determined. It should be noted that such ratio was not easily determined; rather, it required investigation and discovery in order to arrive at the desired localized efficacy and effectiveness specifically tailored for the target tissue. Too low of a drug to polymer ratio is not attractive as local drug concentrations are too low to be effective. Too high of a drug to polymer ratio results in the drug releasing from the SRF too fast, which limits the longer term local drug release and can increase the chances for undesirably high systemic drug exposure. A preferred drug to polymer ratio is 5% to 30% by weight, and more preferred is a 7.5% to 12.5% by weight for optimal drug release and retention.

Following are additional listing of disclosed embodiments:

0.5mL N-methyl pyrrolidone (NMP) was added to a vial with 0.25g sirolimus and vortexed until dissolved. 50 microliter of drug solution was added to 2.5mL of a 50% PLGA8515A (0.3 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 50 microliter of drug solution was added to 2.5mL of a 50% PLGA8515A (0.5 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 50 microliter of drug solution was added to 2.5mL of a 50% PLGA8515E (0.3 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 50 microliter of drug solution was added to 2.5mL of a 50% PLGA8515E (0.5 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 50 microliter of drug solution was added to 2.5mL of a 50% PLGA8515A (0.7 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 50 microliter of drug solution was added to 2.5mL of a 50% PLGA8515E (0.7 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 100 microliter of drug solution was added to 2.5mL of a 50% PLGA8515A (0.3 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 100 microliter of drug solution was added to 2.5mL of a 50% PLGA8515E (0.3 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 100 microliter of drug solution was added to 2.5mL of a 50% PLGA8515A (0.5 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 100 microliter of drug solution was added to 2.5mL of a 50% PLGA8515E (0.7 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 100 microliter of drug solution was added to 2.5mL of a 50% PLGA8515E (0.3 dl/g) in NMP solution using syringe to syringe mixing with a female to female luer connector. 500 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 100 microliter of drug solution was added to 2.5mL of a 50% PLGA8515A (0.5 dl/g) in NMP solution using syringe to syringe mixing with a female to female luer connector. 500 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 100 microliter of drug solution was added to 2.5mL of a 50% PLGA8515E (0.7 dl/g) in NMP solution using syringe to syringe mixing with a female to female luer connector. 500 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 200 microliter of drug solution was added to 2.5mL of a 50% PLGA8515 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 50 microliter of drug solution was added to 2.5mL of a 50% PLGA7525 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 100 microliter of drug solution was added to 2.5mL of a 50% PLGA7525 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 200 microliter of drug solution was added to 2.5mL of a 50% PLGA7525 in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g sirolimus and vortexed until dissolved. 50 microliter of drug solution was added to 2.5mL of a 50% PLGA5050 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 100 microliter of drug solution was added to 2.5mL of a 50% PLGA5050in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g sirolimus and vortexed until dissolved. 200 microliter of drug solution was added to 2.5mL of a 50% PLGA5050 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 50 microliter of drug solution was added to 2.5mL of a 50% PLGA8515A (0.3 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 50 microliter of drug solution was added to 2.5mL of a 50% PLGA8515E (0.3 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 50 microliter of drug solution was added to 2.5mL of a 50% PLGA8515A (0.5 dl/g) in NMP solution using syringe to syringe mixing with a female to female luer connector. 250 microliters in a 1mL syringe was loaded into a 20 G x 20 cm Chiba biopsy needle with depth markers.0.5mL N-methyl pyrrolidone (NMP) was added to a vial with 0.25g everolimus and vortexed until dissolved. 50 microliter of drug solution was added to 2.5mL of a 50% PLGA8515E (0.3 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 50 microliter of drug solution was added to 2.5mL of a 50% PLGA8515A (0.5 dl/g) in NMP solution using syringe to syringe mixing with a female to female luer connector. 500 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 50 microliter of drug solution was added to 2.5mL of a 50% PLGA8515E (0.7 dl/g) in NMP solution using syringe to syringe mixing with a female to female luer connector. 500 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 50 microliter of drug solution was added to 2.5mL of a 50% PLGA8515E (0.5 dl/g) in NMP solution using syringe to syringe mixing with a female to female luer connector. 800 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 50 microliter of drug solution was added to 2.5mL of a 50% PLGA8515A (0.7 dl/g) in NMP solution using syringe to syringe mixing with a female to female luer connector. 800 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 50 microliter of drug solution was added to 2.5mL of a 50% PLGA8515A (0.7 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 50 microliter of drug solution was added to 2.5mL of a 50% PLGA8515E (0.7 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 100 microliter of drug solution was added to 2.5mL of a 50% PLGA8515 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 200 microliter of drug solution was added to 2.5mL of a 50% PLGA8515 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 50 microliter of drug solution was added to 2.5mL of a 50% PLGA7525 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 100 microliter of drug solution was added to 2.5mL of a 50% PLGA7525 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 200 microliter of drug solution was added to 2.5mL of a 50% PLGA7525A (0.3 dl/g) in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 200 microliter of drug solution was added to 2.5mL of a 50% PLGA7525E (0.3 dl/g) in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 200 microliter of drug solution was added to 2.5mL of a 50% PLGA7525A (0.5 dl/g) in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 200 microliter of drug solution was added to 2.5mL of a 50% PLGA7525E (0.5 dl/g) in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 200 microliter of drug solution was added to 2.5mL of a 50% PLGA7525A (0.7 dl/g) in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 200 microliter of drug solution was added to 2.5mL of a 50% PLGA7525E (0.7 dl/g) in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 200 microliter of drug solution was added to 2.5mL of a 50% PLGA6535E (0.3 dl/g) in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 200 microliter of drug solution was added to 2.5mL of a 50% PLGA6535A (0.3 dl/g) in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 200 microliter of drug solution was added to 2.5mL of a 50% PLGA6535E (0.5 dl/g) in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 200 microliter of drug solution was added to 2.5mL of a 50% PLGA6535A (0.5 dl/g) in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 200 microliter of drug solution was added to 2.5mL of a 50% PLGA6535E (0.5 dl/g) in NMP solution using syringe to syringe mixing with a female to female luer connector. 500 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 200 microliter of drug solution was added to 2.5mL of a 50% PLGA6535A (0.5 dl/g) in NMP solution using syringe to syringe mixing with a female to female luer connector. 500 microliters in a 1mL syringe was loaded into a 20 G x 20 cm Chiba biopsy needle with depth markers

0.5mL N-methyl pyrrolidone (NMP) was added to a vial with 0.25g everolimus and vortexed until dissolved. 50 microliter of drug solution was added to 2.5mL of a 50% PLGA5050 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 100 microliter of drug solution was added to 2.5mL of a 50% PLGA5050 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g everolimus and vortexed until dissolved. 200 microliter of drug solution was added to 2.5mL of a 50% PLGA5050 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 50 microliter of drug solution was added to 2.5mL of a 50% PLGA8515 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 100 microliter of drug solution was added to 2.5mL of a 50% PLGA8515A (0.3 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 100 microliter of drug solution was added to 2.5mL of a 50% PLGA8515E (0.3 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 100 microliter of drug solution was added to 2.5mL of a 50% PLGA8515A (0.5 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 100 microliter of drug solution was added to 2.5mL of a 50% PLGA8515E (0.5 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 100 microliter of drug solution was added to 2.5mL of a 50% PLGA8515A (0.7 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 100 microliter of drug solution was added to 2.5mL of a 50% PLGA8515E (0.7 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 100 microliter of drug solution was added to 2.5mL of a 50% PLGA8515A (0.5 dl/g) in NMP solution using syringe to syringe mixing with a female to female luer connector. 500 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 100 microliter of drug solution was added to 2.5mL of a 50% PLGA8515E (0.5 dl/g) in NMP solution using syringe to syringe mixing with a female to female luer connector. 500 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 100 microliter of drug solution was added to 2.5mL of a 50% PLGA8515A (0.7 dl/g) in NMP solution using syringe to syringe mixing with a female to female luer connector. 500 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 500 microliter of drug solution was added to 2.5mL of a 50% PLGA8515E (0.7 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 200 microliter of drug solution was added to 2.5mL of a 50% PLGA8515 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 50 microliter of drug solution was added to 2.5mL of a 50% PLGA7525 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 100 microliter of drug solution was added to 2.5mL of a 50% PLGA7525 in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 200 microliter of drug solution was added to 2.5mL of a 50% PLGA7525 in NMP solution using syringe to syringe mixing with a female to female luer connector. 250 microliters in a 1mL syringe was loaded into a 20 G x 20 cm Chiba biopsy needle with depth markers

0.5mL N-methyl pyrrolidone (NMP) was added to a vial with 0.25g paclitaxel and vortexed until dissolved. 100 microliter of drug solution was added to 2.5mL of a 50% PLGA6535A (0.3 dl/g) in NMP solution using syringe to syringe mixing with a female to female luer connector. 500 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 100 microliter of drug solution was added to 2.5mL of a 50% PLGA6535A (0.5 dl/g) in NMP solution using syringe to syringe mixing with a female to female luer connector. 500 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 500 microliter of drug solution was added to 2.5mL of a 50% PLGA6535E (0.3 dl/g) in NMP solution using syringe to syringe mixing with a female to female luer connector. 250 microliters in a 1mL syringe was loaded into a 20 G x 20 cm Chiba biopsy needle with depth markers.

0.5mL N-methyl pyrrolidone (NMP) was added to a vial with 0.25g paclitaxel and vortexed until dissolved. 500 microliter of drug solution was added to 2.5mL of a 50% PLGA6535E (0.5 dl/g) in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.25g paclitaxel and vortexed until dissolved. 400 mg of drug solution was added to 2.9g of a 20% by weight PLGA5050A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 30% by weight PLGA5050A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA5050A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA5050A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA5050A in NMP solution using syringe to syringe mixing with a female to female luer connector (#SRF1). 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA5050A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.25g paclitaxel and vortexed until dissolved. 400 mg of drug solution was added to 2.9g of a 20% by weight PLGA5050E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 30% by weight PLGA5050E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA5050E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA5050E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA5050E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA5050E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.25g paclitaxel and vortexed until dissolved. 400 mg of drug solution was added to 2.9g of a 20% by weight PLGA8515A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 30% by weight PLGA8515A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA8515A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA8515A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA8515A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA8515A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.25g paclitaxel and vortexed until dissolved. 400 mg of drug solution was added to 2.9g of a 20% by weight PLGA8515E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 30% by weight PLGA8515E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA8515E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA8515E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA8515E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA8515E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.25g paclitaxel and vortexed until dissolved. 400 mg of drug solution was added to 2.9g of a 20% by weight PLGA6535A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 30% by weight PLGA6535A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA6535A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA6535A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA5050A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA6535A in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.25g paclitaxel and vortexed until dissolved. 400 mg of drug solution was added to 2.9g of a 20% by weight PLGA6535E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 30% by weight PLGA6535E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA6535E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 40% by weight PLGA6535E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA6535E in NMP solution using syringe to syringe mixing with a female to female luer connector. 250 microliters in a 1mL syringe was loaded into a 20 G x 20 cm Chiba biopsy needle with depth markers.

1.08g N-methyl pyrrolidone (NMP) was added to a vial with 0.50g paclitaxel and vortexed until dissolved. 400 mg of drug solution was added to 2.9g of a 50% by weight PLGA6535E in NMP solution using syringe to syringe mixing with a female to female luer connector. 250 microliters in a 1mL syringe was loaded into a 20 G x 20 cm Chiba biopsy needle with depth markers.

## Claims

1. A sustained release formulation (SRF) for use in a method of treatment of benign prostatic hyperplasia (BPH), the SRF comprising a cytostatic or cytotoxic drug,
   wherein the method comprises dispensing the SRF into the target tissue of the prostate and wherein dispensing comprises 10-500 microliter per injection over 1-10 injections, across each side of the prostate.

2. The SRF for use of claim 1, wherein the SRF comprises a glycolide-based bioabsorbable copolymer.

3. The SRF for use of claim 2, wherein the SRF includes a non-toxic, water-soluble solvent capable of dissolving the copolymer and the cytostatic or cytotoxic drug.

4. The SRF for use of claim 1, wherein dispensing comprises 10-50 microliter per injection, 50-100 microliter per injection, 100-300 microliter per injection or 200-500 microliter per injection over 2 or 3 injections.

5. The SRF for use of claim 4, wherein dispensing comprises 100 microliter per injection.

6. The SRF for use of any preceding claim, wherein the SRF is adapted to release the drug into the prostate over at least 14 days from being injected into the prostate, or over a 30 to 90 day period, or over a 90 to 180 day period.

7. The SRF for use of any preceding claim, wherein the SRF comprises glycolide-based bioabsorbable copolymer at a concentration of 25 - 75% by wt. of the bioabsorbable copolymer, 75-25% by wt. of solvent and 0.5% - 30% by wt. drug; 1% - 20% by wt. of drug, or 1% - 5% by wt. of drug.

8. The SRF for use of claim 1, wherein the drug has a release rate of no more than 10% to 75% over the first month, 25% to 95% over the first three months, and/or 50% to 100% over the first six months.

9. The SRF for use of claim 1, wherein the drug has a release rate of between 5% to 50% during the first 24 hours from injecting the SRF into the prostate.

10. The SRF for use of claim 1, wherein the SRF comprises DL-lactide glycolide bioabsorbable copolymer and the inherent viscosity of the copolymer is between 0.1 - 0.6 dL/g, 0.1 to 0.4 dL/g or 0.2 to 0.3 dL/g and the ratio of DL-lactide to glycolide is from 50/50 up to 90/10, 95/5, or 85/15.

11. The SRF for use of claim 1, wherein the cytostatic or cytotoxic drug is 0.1 up to 10 % by wt. or up to 20-30% by wt. of the SRF.

12. The SRF for use of claim 1, wherein dispensing comprises 10-200 microliter per injection over 1-10 injections, or 200-500 microliter per injection over 1-5 injections, across each side of the prostate.

**Patentansprüche**

1. Retardformulierung (Sustained Release Formulation, SRF) zur Verwendung bei einem Verfahren zur Behandlung von gutartiger Prostatahyperplasie (BPH), wobei die SRF ein Zytostatikum oder einen zytotoxischen Arzneistoff umfasst,
wobei das Verfahren Dosieren der SRF in das Zielgewebe der Prostata umfasst und wobei das Dosieren 10-500 Mikroliter pro Injektion über 1-10 Injektionen über jede Seite der Prostata umfasst.

2. SRF zur Verwendung nach Anspruch 1, wobei die SRF ein bioabsorbierbares Copolymer auf Glycolidbasis umfasst.

3. SRF zur Verwendung nach Anspruch 2, wobei die SRF ein nichttoxisches, wasserlösliches Lösungsmittel enthält, das das Copolymer und das Zytostatikum bzw. den zytotoxischen Arzneistoff lösen kann.

4. SRF zur Verwendung nach Anspruch 1, wobei das Dosieren 10-50 Mikroliter pro Injektion, 50-100 Mikroliter pro Injektion, 100-300 Mikroliter pro Injektion oder 200-500 Mikroliter pro Injektion über 2 oder 3 Injektionen umfasst.

5. SRF zur Verwendung nach Anspruch 4, wobei das Dosieren 100 Mikroliter pro Injektion umfasst.

6. SRF zur Verwendung nach einem vorhergehenden Anspruch, wobei die SRF so ausgelegt ist, dass der Arzneistoff über mindestens 14 Tage nach Injektion in die Prostata oder über einen Zeitraum von 30 bis 90 Tagen oder über einen Zeitraum von 90 bis 180 Tagen in die Prostata freigesetzt wird.

7. SRF zur Verwendung nach einem vorhergehenden Anspruch, wobei die SRF bioabsorbierbares Copolymer auf Glycolidbasis in einer Konzentration von 25 - 75 Gew.-% bioresorbierbares Copolymer, 75 - 25 Gew.-%. Lösungsmittel und 0,5 - 30 Gew.-%. Arzneistoff, 1 bis 20 Gew.-% Arzneistoff oder 1 - 5 Gew.-% Arzneistoff umfasst.

8. SRF zur Verwendung nach Anspruch 1, wobei der Arzneistoff eine Freisetzungsrate von nicht mehr als 10 % bis 75 % über den ersten Monat, 25 % bis 95 % über die ersten drei Monate und/oder 50 % bis 100 % über die ersten sechs Monate aufweist.

9. SRF zur Verwendung nach Anspruch 1, wobei der Arzneistoff eine Freisetzungsrate zwischen 5 % und 50 % während der ersten 24 Stunden nach Injizieren der SRF in die Prostata aufweist.

10. SRF zur Verwendung nach Anspruch 1, wobei die SRF bioabsorbierbares DL-Lactid-Glycolid-Copolymer umfasst und die inhärente Viskosität des Copolymers bei 0,1 - 0,6 dl/g, 0,1 bis 0,4 dl/g oder 0,2 bis 0,3 dl/g liegt und das Verhältnis von DL-Lactid zu Glycolid bei 50:50 bis zu 90:10, 95:5 oder 85:15 liegt.

11. SRF zur Verwendung nach Anspruch 1, wobei das Zytostatikum bzw. der zytotoxische Arzneistoff bei 0,1 bis zu 10 Gew.-% oder bis zu 20-30 Gew.-% der SRF liegt.

12. SRF zur Verwendung nach Anspruch 1, wobei das Dosieren 10-200 Mikroliter pro Injektion über 1-10 Injektionen oder 200-500 Mikroliter pro Injektion über 1-5 Injektionen über jede Seite der Prostata umfasst.

**Revendications**

1. Formulation à libération prolongée (SRF) destinée à être utilisée dans un procédé de traitement de l'hyperplasie bénigne de la prostate (HBP), le SRF comprenant un médicament cytostatique ou cytotoxique,
le procédé comprenant la distribution du SRF dans le tissu cible de la prostate et la distribution comprenant 10-500 microlitres par injection sur 1-10 injections, sur chaque côté de la prostate.

2. SRF pour utilisation selon la revendication 1, le SRF comprenant un copolymère bioabsorbable à base de glycolide.

3. SRF pour utilisation selon la revendication 2, le SRF comprenant un solvant hydrosoluble non toxique capable de dissoudre le copolymère et le médicament cytostatique ou cytotoxique.

4. SRF pour utilisation selon la revendication 1, la distribution comprenant 10-50 microlitres par injection, 50-100 microlitres par injection, 100-300 microlitres par injection ou 200-500 microlitres par injection sur 2 ou 3 injections.

5. SRF pour utilisation selon la revendication 4, la distribution comprenant 100 microlitres par injection.

6. SRF pour utilisation selon l'une quelconque des revendications précédentes, le SRF étant adapté pour libérer le médicament dans la prostate pendant au moins 14 jours après son injection dans la prostate, ou pendant une période de 30 à 90 jours, ou pendant une période de 90 à 180 jours.

7. SRF pour utilisation selon l'une quelconque des revendications précédentes, le SRF comprenant un copolymère bioabsorbable à base de glycolide à une concentration de 25 à 75 % en poids du copolymère bioabsorbable, 75 à 25 % en poids de solvant et 0,5 % à 30 % en poids médicament ; 1 % à 20 % en poids de médicament, soit 1 % à 5 % en poids de médicament.

8. SRF pour utilisation selon la revendication 1, le médicament ayant un taux de libération de pas plus de 10 % à 75 % au cours du premier mois, de 25 % à 95 % au cours des trois premiers mois, et/ou de 50 % à 100 % au cours des six premiers mois.

9. SRF pour utilisation selon la revendication 1, le médicament ayant un taux de libération compris entre 5 % et 50 % pendant les premières 24 heures à partir de l'injection du SRF dans la prostate.

10. SRF pour utilisation selon la revendication 1, le SRF comprenant un copolymère bioabsorbable de glycolide DL-lactide et la viscosité inhérente du copolymère étant comprise entre 0,1 à 0,6 dL/g, 0,1 à 0,4 dL/g ou 0,2 à 0,3 dL/g et le rapport de DL-lactide au glycolide étant compris entre 50/50 et 90/10, 95/5 ou 85/15.

11. SRF pour utilisation selon la revendication 1, le médicament cytostatique ou cytotoxique étant de 0,1 à 10 % en poids, soit jusqu'à 20-30 % en poids, du SRF.

12. SRF pour utilisation selon la revendication 1, la distribution comprenant 10-200 microlitres par injection sur 1-10 injections, ou 200-500 microlitres par injection sur 1-5 injections, sur chaque côté de la prostate.

FIG. 1

FIG. 2

**FIG. 3**

**FIG. 4**

FIG. 5

**FIG. 6A**

FIG. 6B

## FIG. 7

Systemic drug concentrations measured by LCMS as a function of time post SRF-1 injection per animal

Systemic drug mean concentrations for two different doses (5 and 10 mg) measured by LCMS as a function of time post SRF-1 injection per animal

FIG. 8

FIG. 9

**EP 4 216 926 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63081865
- US 09207920
- US 6277391 B1

### Non-patent literature cited in the description

- **ARASH M. SAEMI** ; **JEFFREY B. FOLSOM** ; **MARK K. PLANTE**. Injection therapy for prostatic disease: A renaissance concept. *Indian J Urol.*, July 2008, vol. 24 (3), 329-335
- **XIA, B.-W. et al.** Relationship between serum total testosterone and prostate volume in aging men. *Scientific Reports*, 2021, vol. 11, 14122